# FASCICULE DE BREVET EUROPEEN

(11) **EP 1 721 145 B1**
(45) Date de publication et mention de la délivrance du brevet: **27.12.2017**
(21) Numéro de dépôt: 05736901.9
(22) Date de dépôt: 04.03.2005
(51) Int. Cl.: A61B 5/00, A61B 7/00, G01N 19/02

(54) **SONDE TRIBO ACOUSTIQUE**
TRIBOAKUSTISCHER SENSOR
TRIBOACOUSTIC SENSOR

(30) Priorité: 04.03.2004 FR 0402283
(43) Date de publication de la demande: 15.11.2006
(73) Titulaire: CENTRE NATIONAL DE LA RECHERCHE SCIENTIFIQUE -CNRS-, 75794 Paris Cedex 16 (FR)
(72) Inventeur: ZAHOUANI, Hassan, F-25000 Besançon (FR); VARGIOLU, Roberto, F-69390 Millery (FR); MAVON, Alain, F-31450 Corronsac (FR)
(74) Mandataire: Regimbeau
(86) Numéro de dépôt international: PCT/FR2005/000526
(87) Numéro de publication internationale: WO 2005/085805

(56) Documents cités:
- DE-A- 10 164 044
- FR-A- 2 811 764
- US-A- 5 679 883
- US-A- 5 852 232
- US-A1- 2002 173 223
- "Biomedical micro-tribometer application for skin studies (both in-vivo and in-vitro)" ARTICLE, [Online] 23 août 2003 (2003-08-23), XP002293582 Extrait de l'Internet: URL:www.cetr.com/skin_testing.html> [extrait le 2004-08-12]
- STEVEN T. PATTON, JEFFREY S. ZABINSKI: "Advanced tribometer for in situ studies of friction, wear, and contact condition-Advanced tribometer for friction and wear studies" TRIBOLOGY LETTERS, vol. 13, no. 4, 2002, pages 263-273, XP002337318

## Description

La présente invention concerne le domaine des dispositifs de mesure d'état de surface. Elle concerne plus particulièrement une sonde pour mesurer les propriétés acoustiques et tribologiques (appelées propriétés tribo-acoustiques dans la suite de l'exposé) et ainsi quantifier le toucher d'une surface. Elle trouve son application pour la mesure des propriétés tribo-acoustiques de la peau et des phanères, de tissus, du cuir, des matériaux plastiques, ou de tout autre matériau pour lequel l'appréciation du toucher est importante.

On entend par toucher les qualités tactiles d'un matériau, c'est-à-dire sa douceur, sa fermeté, son élasticité, sa finesse, sa résilience et autres qualités perceptible par le toucher. Cette notion, pour les besoins industriels, est essentiellement mesurée par appréciations tactiles et subjectives sur panels. Ce sont donc des experts qui après formation se prononcent qualitativement sur l'appréciation du toucher. C'est notamment le cas lorsqu'il s'agit d'évaluer l'impact en dermatologie d'une crème appliquée sur la peau.

Ces appréciations correspondent en fait à l'évaluation in vivo des propriétés tribologiques (contact, frottement) et acoustiques de la surface en question.

On comprend bien alors le caractère aléatoire et très subjectif de cette approche, car elle reste très dépendante de l'expert.

Le document DE 101 64 044 concerne une méthode de mesure de la dureté de tissu dentaire comme les amalgames ou les matériaux prothétiques. Cette méthode utilise le bruit généré par un objet dur comme une pointe arrondie, par exemple sphérique, lors de son déplacement sur les tissus en question.

Le document « Biomedical micro-tribometer application for skin sutides », 23 août 2003, XP002293582, concerne l'utilisation d'une sonde pour mesurer les propriétés de la peau à travers la quantification de son coefficient de frottement, et de ses propriétés électriques. La mesure du coefficient de frottement comprend notamment la mesure du coefficient statique ainsi que celle du coefficient dynamique. Le tribomètre divulgué mesure notamment la force normale ainsi que la force de frottement simultanément lors d'un déplacement de cette sonde.

Le document « Advanced tribometer for in situ studies of friction, wear, and contact condition - Advanced tribometer for friction and wear studies », Tribology Letters vol. 13, n° 4 2002, p 263-279, concerne un tribomètre qui associe des mesures de friction, usure, force de friction cinétique, résistance de contact, émission acoustique à l'aide d'un transducteur piézo-électrique pour contrôle du contact et réflectance de surface.

Le but de la présente invention est de proposer une sonde permettant de quantifier et de caractériser le toucher via l'acquisition de données physiques, comme les forces de frottement statique et dynamique, et les ondes sonores.

A cet effet, la présente invention a pour objet une sonde pour la mesure quantitative du toucher d'une surface, comprenant :
- une enveloppe préhensible,
- un corps de contact destiné à être en contact avec la surface sur une zone de sondage,
- des éléments de détection acoustique pour détecter des bruits émis par le corps lors de son contact avec la zone de sondage,
- caractérisée en ce que le corps de contact (6) est un corps creux, que les éléments de détection acoustique (5) sont des premiers éléments de détection placés dans le corps creux et la sonde comprenant en outre des deuxièmes éléments de détection mécaniques agencés pour mesurer l'effort normal et l'effort de frottement exercés par la surface sur le corps creux.

Ainsi la sonde assure, par un balayage sur la zone du corps ou de la surface à étudier, la mesure du comportement mécano-acoustique de cette surface grâce à la quantification de paramètres spécifiques.

Avantageusement, les premiers éléments de détection acoustique comprennent un microphone maintenu dans l'enveloppe préhensible, ce microphone comprenant une membrane localisée à l'intérieur du corps creux.

Par ailleurs, les deuxièmes éléments de détection mécaniques comprennent respectivement au moins un capteur d'effort normal adapté pour mesurer l'effort normal et au moins un capteur d'effort de frottement adapté pour mesurer l'effort de frottement, efforts subis par le corps creux, lors de son contact avec la zone de sondage.

Dans un mode de réalisation préféré, le corps creux est de forme sphérique. Avantageusement, il est réalisé dans un matériau présentant d'excellentes capacités de résonance, et un minimum de rigidité, comme notamment une fibre de carbone.

D'autres caractéristiques et avantages de l'invention apparaîtront encore à la lecture de la description qui va suivre. Celle-ci est purement illustrative et doit être lue en regard des dessins annexés sur lesquels :
- la figure la est une vue d'ensemble d'un mode de réalisation de la sonde selon l'invention,
- la figure 1b est une vue de dessus de la sonde de la figure la,
- la figure 2 est une vue en coupe II-II de la sonde de la figure la,
- la figure 3 est une vue de la pièce de forme allongée destinée à la transmission des efforts dans la sonde des figures la, 1b et 2,
- la figure 4 est une vue d'ensemble de la sonde de la figure 1 et d'une unité électronique de calcul lors d'une mesure sur une surface,
- la figure 5a est un schéma de la sonde dans un second mode de réalisation,
- la figure 5b est un schéma de la sonde dans un troisième mode de réalisation.

Un exemple de réalisation d'une sonde selon l'invention est représenté aux figures la et 1b.

La sonde représentée à la figure la, est constituée d'une enveloppe externe 1, par exemple métallique. Elle peut être de forme cylindrique et allongée de manière à être facilement préhensible par l'opérateur. L'enveloppe externe 1 est obturée à une de ses extrémités par un corps de maintien 2 qui s'étend à l'intérieur de l'enveloppe externe 1. Du corps de maintien 2, constitué d'un alliage métallique, s'échappent des fils électriques 12 de transmission de données vers une unité électronique de calcul (non représentée). A l'autre extrémité 14 de l'enveloppe externe 1, on trouve l'élément frottant 6 de la sonde destiné à être appliqué et déplacé sur la surface à analyser. Une diode laser 9 est placée près de la tête de l'enveloppe externe 1. Cette diode laser 9 permet de tracer un segment de droite, indiquant le sens de frottement de la sonde, sur la surface à analyser. La L'enveloppe externe 1 peut avantageusement être peinte afin de minimiser l'influence des bruits environnants.

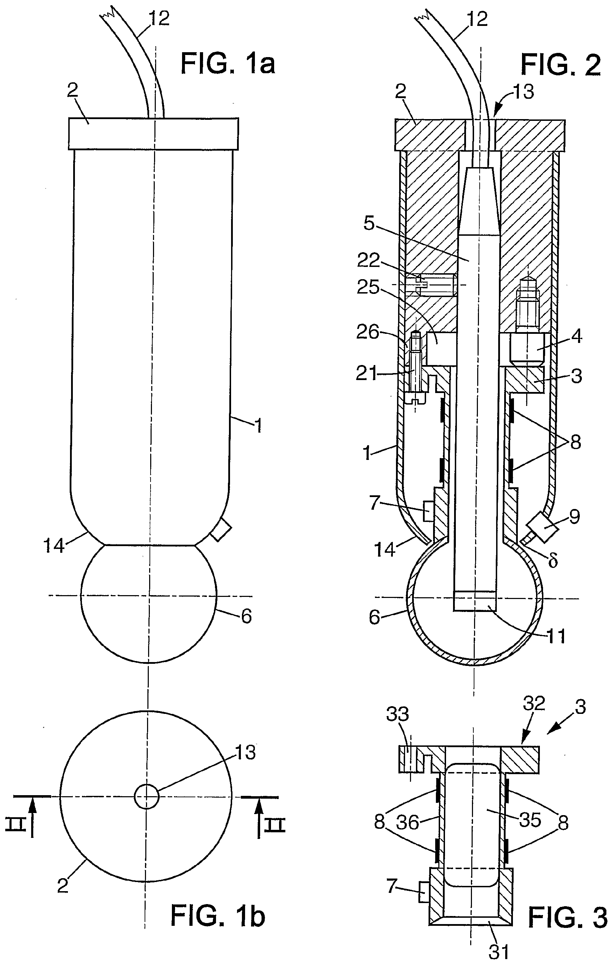

La figure 1b présente une vue de dessus de l'extrémité de la sonde côté fils électriques 12. On retrouve le corps de maintien 2 ainsi que la sortie 13 des fils électriques 12 de transmission vers l'unité électronique de calcul.

La figure 2 représente une vue en coupe II-II de la sonde représentée à la figure la. Le corps de maintien 2 est de forme cylindrique et vient épouser la surface interne de l'enveloppe externe 1. Une ouverture est aménagée à l'intérieur du corps de maintien 2 afin qu'il puisse contenir en partie un microphone 5. Cette ouverture se prolonge dans la partie extérieure du corps de maintien 2 par la sortie 13 aménagée pour les fils électriques 12.

Le microphone 5 est retenu à l'intérieur du corps de maintien 2 via une vis de maintien 22 prise dans le corps de maintien et serrée sur microphone 5. Le microphone 5 est de forme allongée, et son autre extrémité, la tête du microphone 11 comportant une membrane vibrante acoustique, est placée à l'intérieur de l'élément frottant 6 qui apparaît, à la figure 2, comme un corps creux, préférablement sphérique.

Le corps creux 6 est fixé sur une pièce de forme allongée 3. Cette pièce de forme allongée 3 dans la même direction que l'enveloppe 1 est fixée sur le corps de maintien 2 par l'intermédiaire d'une vis de maintien 21 à l'intérieur de l'enveloppe externe 1. La pièce de forme allongée s'étend depuis le corps de maintien 2 jusqu'à l'extrémité 14 de l'enveloppe externe 1. La longueur de la pièce de forme allongée 3 est telle qu'un jeu δ subsiste entre l'extrémité 14 de l'enveloppe externe 1 et le corps creux 6 fixé sur l'extrémité de la pièce de forme allongée. La pièce de forme allongée 3 n'est préférablement fixée sur le corps de maintien 2 que par un seul côté. La fixation est réalisée sur une extrémité dépassante 26 du corps de maintien 2, extrémité de surface réduite par rapport à la section du corps de maintien 2. Sur la figure 2, une vis de maintien 21 est représentée, une deuxième vis symétrique par rapport au plan de coupe n'est pas représentée. Un espace 25 est aménagé entre la pièce de forme allongée 3 et le corps de maintien 2 sur l'essentiel de leurs surfaces en regard. Ainsi, ces deux pièces peuvent fléchir l'une par rapport à l'autre grâce à l'espace 25 et leur fixation de surface réduite.

Dans cet espace 25 est placé un capteur d'effort normal 4 dont la partie fixe est maintenue sur le corps de maintien 2 et dont la partie mobile est en contact avec la pièce de forme allongée 3. Ce capteur d'effort normal 4 est ainsi capable, lors du déplacement de la sonde sur une surface à étudier, de détecter tout effort normal appliqué par la surface à sonder sur le corps creux 6. En effet, le jeu δ entre le corps creux 6 et l'extrémité 14 de l'enveloppe externe 1 d'une part, ainsi que le fléchissement possible entre la pièce de forme allongée 3 et le corps de maintien 2 d'autre part, assurent la transmission de l'effort normal depuis la surface à sonder jusqu'au la partie mobile du capteur 4.

Un accéléromètre 7 est placé latéralement sur la pièce de forme allongée 3 à proximité du corps creux 6. Des jauges de contraintes 8, au nombre de 4 sur la figure 2, sont fixés sur la surface extérieure de la pièce de forme allongée 3. L'accéléromètre 7 et les jauges de contraintes 8 constituent des capteurs de l'effort de frottement appliqué au corps creux 6. La forme de la pièce de forme allongée 3 ainsi que le jeu δ autorise la flexion de cette pièce lors du déplacement du corps creux 6 de la sonde au contact de la surface à sonder. Les déplacements tangentiels du corps creux par rapport à l'enveloppe externe 1 sont ainsi permis. Cette flexion est directement mesuré par l'accéléromètre et les jauges de contraintes 8.

Des fils électriques (non représentés) relient les différents capteurs aux fils électriques 12 de transmission vers une unité électronique de calcul.

Une ouverture est aménagée au centre de la pièce de forme allongée 3 afin de laisser passer le corps du microphone 5. Cette ouverture a un diamètre supérieur aux dimensions externes du microphone afin que la pièce de forme allongée 3 ne vienne entrer en contact avec le corps du microphone au cours de ces déformations.

La figure 3 représente la pièce de forme allongée 3 munie des jauges de contraintes 8 et de l'accéléromètre 7. On retrouve l'ouverture aménagée en son centre, le long de son axe afin de laisser passer le corps du microphone. Deux ouvertures 35 sont aménagées de part et d'autre de la pièce de forme allongée dans sa grande longueur, afin que subsistent deux lames 36 formées de part et d'autre de la pièce 3. Ce sont ces lames 36 qui portent les jauges de contraintes 8. L'épaisseur des lames 36 est calculée en fonction des caractéristiques du matériau constituant la pièce 3 et des jauges de contrainte 8. Sur l'exemple de la figure 3, chaque lame 36 porte deux jauges de contrainte. La pièce 3 est usinée d'un seul tenant ; elle est évidée à la fois en son centre pour laisser passer le corps du microphone et sur les côtés pour créer les lames supportant les jauges de contraintes. Les ouvertures 35 sont préférentiellement évidées de manière à ce que les jauges de contrainte 8, et la diode laser 9 de la figure 2, appartiennent sensiblement au même plan. La flexion de la pièce de forme allongée 3 est ainsi facilitée grâce aux ouvertures 35, lors du déplacement de l'élément frottant 6 sur une surface à analyser, dans la direction pointée par la diode laser 9.

Sur une des extrémités 31 de la pièce de forme allongée est aménagé un trou conique destiné à la fixation du corps creux 6. Sur son extrémité opposée, un filetage 33 est aménagé pour venir serrer la vis de maintien 21 sur le corps de maintien 2. Diamétralement opposé au filetage 33, sur cette même extrémité de la pièce de forme allongée 3, on retrouve la surface d'appui 32 en contact avec la partie mobile du capteur d'effort normal 4. On peut bien entendu envisager le cas inverse où le capteur d'effort normal est fixé sur la pièce de forme allongée 3 et sa partie mobile s'appuie sur la surface en regard du corps de maintien 2.

Le corps creux 6 constitue l'élément frottant de la sonde. Il contient de l'air libre et doit se comporter en caisse de résonance, afin d'assurer une bonne transmission acoustique des bruits résultant du déplacement du corps creux 6 sur la surface à analyser. Il doit par ailleurs être suffisamment rigide afin de transmettre les efforts normal et de frottement lors de son déplacement sur la surface à sonder. Des matériaux du type fibre de carbone présentent de telles caractéristiques. Une balle de ping-pong, par exemple, constitue un excellent élément frottant pour une sonde selon l'invention.

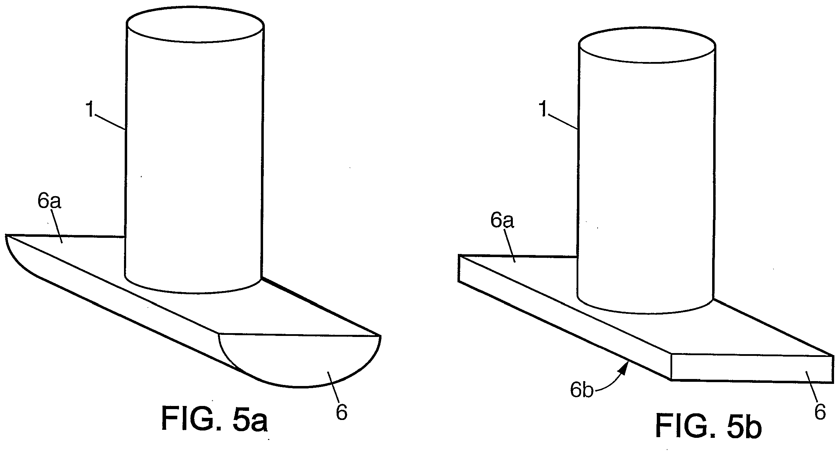

Les figures 5a et 5b présentent respectivement un second et troisième mode de réalisation de la sonde selon l'invention. Le corps creux 6 présente des formes différentes de la forme sphérique de la figure 1. A la figure 1, le corps creux 6 présente une partie supérieur 6a plane, de forme sensiblement rectangulaire, et fixé en son centre sur la pièce de forme allongée 3 (non représenté sur la figure 5a). La partie inférieure 6b du corps creux est formée par une portion de cylindre. Le corps creux 6 présente donc la forme d'un portion de cylindre issue de la coupe d'un cylindre par un plan parallèle à son axe. La partie arrondie du corps creux est la partie destinée à entrée en contact avec la surface à analyser. Pour ce type de corps creux, le déplacement de la sonde est réalisé dans une direction sensiblement perpendiculaire à l'axe de la portion de cylindre 6b. La surface de contact du corps creux avec la surface à analyser correspond à une surface sur la portion de cylindre sensiblement parallèle à l'axe de cette dernière.

Le corps creux présenté à la figure 5b est de forme sensiblement parallélépipédique, la surface supérieur 6a est de forme similaire à celle de la figure 5a, et le corps creux est fermé par une partie inférieur 6b de manière à former un parallélépipède avec une surface inférieure 6c sensiblement parallèle à la surface supérieure 6a. Ce corps creux offre une large surface de contact avec la surface à analyser.

Le microphone est un microphone classique, de forme allongée, couramment trouvé dans le commerce. Il doit présenter de bonnes capacités acoustiques. Le microphone constitue des premiers éléments de détection acoustique de la sonde selon l'invention.

Le capteur d'effort normal est un capteur de force miniature capable de lire des efforts de zéro à quelques newtons et de réaliser des mesures statiques et dynamiques. Dans un autre mode de réalisation, notamment pour tenir compte des différentes formes que peut prendre le corps creux, comme vu précédemment, le capteur d'effort normal 4 peut avantageusement être remplacé par capteur de pression. Ce dernier présente l'avantage de mesurer la pression normale exercée par la surface à sonder sur le corps creux 6 indépendamment de la forme du corps creux. Le capteur de pression est mis en place de la même manière que le capteur d'effort normal décrit précédemment.

Les jauges de contraintes permettent de déterminer l'effort de frottement statique et quasi statique, alors que l'accéléromètre permet d'accéder à la composante dynamique de ce même effort.

Les capteurs des efforts normal et de frottement constituent des seconds éléments de détection mécanique. La pièce de forme allongée 3 transmet les efforts subis par le corps creux 6 des figures 1a et 1b aux seconds éléments de détection mécanique.

La sonde selon l'invention trouve particulièrement son application dans la mesure de l'impact sur les propriétés tribo-acoustiques d'un traitement appliqué à la surface de sondage. En cosmétologie par exemple, la sonde permet de quantifier l'impact d'un produit hydratant sur la peau, en comparant les propriétés tribo-acoustiques relevées sur une zone test de peau avant toute application, aux propriétés tribo-acoustiques relevées sur cette même zone à intervalles de temps successifs, après application du produit hydratant. Des applications similaires peuvent être envisagées par exemple en quantifiant l'impact d'un shampoing sur un cheveu.

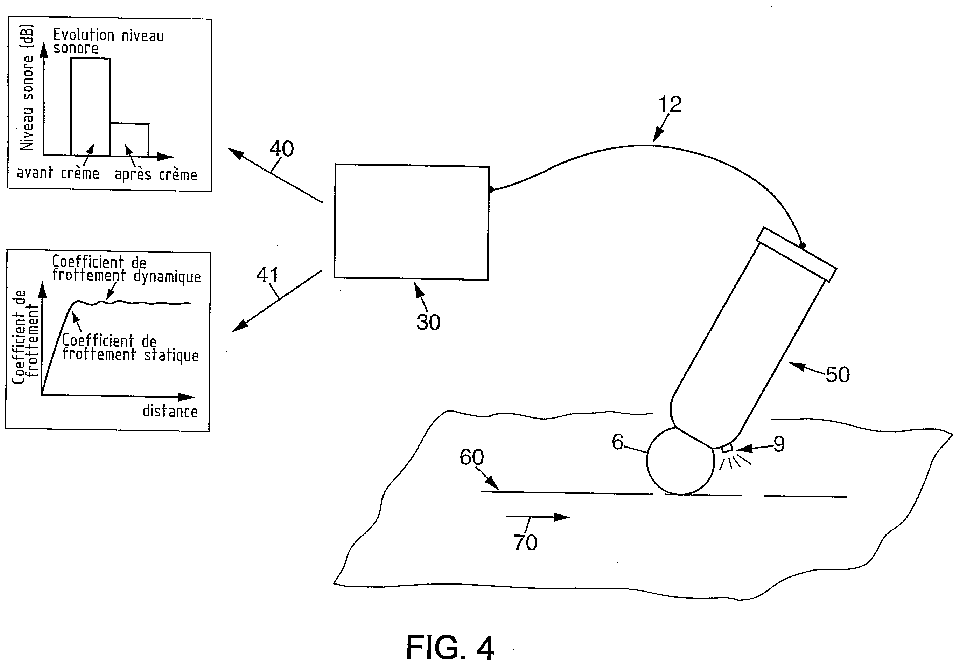

La figure 4 représente une vue d'ensemble de la sonde et d'une unité électronique de calcul lors de son utilisation pour la caractérisation du toucher d'une surface 20. L'opérateur (non représenté sur la figure 4) met en contact l'élément frottant 6 de la sonde 50 sur la zone de sondage de la surface 20 à étudier, et effectue un balayage linéaire de frottement sur celle-ci dans une direction 70 le long d'une ligne 60. La diode laser 9, en traçant un segment de droite visible sur la surface 20, permet à l'opérateur de facilement suivre la ligne 60 et la direction de déplacement 70. Elle permet également lors de passages successifs sur la ligne 60 de repositionner la sonde grâce à des repères tracés par l'opérateur sur la ligne 60.

Dans un autre mode de réalisation, on peut ajouter à la sonde un dispositif de mesure de la vitesse de déplacement sur la surface à analyser. En effet, on peut venir compléter la diode laser 9 par une caméra optique afin un dispositif de mesure pour déterminer la vitesse de déplacement de la sonde sur la surface à analyser. Cette technologie est connue des souris optiques. De telles souris optiques sont décrites dans les brevets US 4,364,035 et 4,390,873. Une autre souris optique a été décrite en détail dans l'article "The Optical Mouse, And An Architectural Methodology For Smart Digital Sensors" Richard F. Lyon, VLSI-81-1 August 1981. Cet mesure de la vitesse permet de contrôler la vitesse de déplacement de la sonde et ainsi assurer un bon étalonnage de l'instrument. L'opérateur peut également contrôler la vitesse de déplacement de la sonde. On peut également envisager de corriger les valeurs mesurées en fonction de la vitesse de déplacement pour rendre les mesures indépendantes de l'utilisateur.

L'ensemble des données relevées par le microphone d'une part, et par les capteurs des efforts normal et de frottement d'autre part, (et le cas échéant par le dispositif de mesure de la vitesse lorsqu'il est prévu) est transmis par les fils électriques 12 de transmission vers une unité électronique de calcul 30. Les données obtenues sont alors traitées par des algorithmes de calculs complexes qui permettent d'obtenir des paramètres simples de quantification des propriétés acoustiques et tribologiques de la surface à étudier. L'unité électronique 30 peut également émettre une information qualitative du type son liée à l'amplitude des données lues afin que l'opérateur puisse combiner les résultats calculés à une appréciation subjective.

En ce qui concerne le traitement du signal acoustique, lors du balayage linéaire de frottement le long de la ligne 60 par l'opérateur, le bruit est amplifié par les capacités de résonance du corps creux, et est capté un préamplificateur monté derrière la membrane du microphone (non représenté sur la figure 4) pour être transformé en signal électrique représentatif du signal sonore. Les fils électriques de transmission 12 acheminent le signal électrique ainsi capté vers l'unité électronique 30 de calcul.

L'information sonore de la figure 4 peut être traitée par exemple par transformation de Fourier d'une part et par décomposition en ondelettes continues. La transformation de Fourier permet le calcul de la densité spectrale de puissance de base du signal sonore. Elle permet aussi de rendre compte de la multitude de phénomènes physiques et physiologiques en jeu à l'interface entre l'élément frottant de la sonde et la surface à analyser. Elle permet aussi d'accéder au niveau sonore moyen en décibels à partir du spectre issu de la transformation qui présente le double intérêt de replacer les mesures sur une échelle universellement appréhendable et de représenter l'énergie diffusée lors du frottement de l'élément frottant sur la surface à analyser. L'analyse par ondelettes continues, quant à elle, permet de représenter le signal sonore suivant une base temps-fréquence.

Ces différents paramètres calculés à partir du signal sonore peuvent permettre de quantifier et de qualifier in vivo l'effet (rémanence, biodisponibilité, ...) de l'adjonction d'actifs sur des surfaces telles que la peau ou un cheveu par exemple. On peut notamment constater une chute des niveaux sonores, comme représenté sur le graphe 40 de la figure 4, niveaux lus après application d'une crème réparatrice sur la peau.

En ce qui concerne le traitement des informations recueillies par les capteurs d'effort normal (ou le capteur de pression normale) et le capteur de frottement, les signaux électriques lus sont acheminés par les fils électriques de transmission 12 vers l'unité électronique 30 de calcul. Ces signaux peuvent alors être transformés par logiciel en effort normal et effort tangentiel, pour, par exemple, calculer l'évolution du coefficient de frottement en fonction du déplacement.

La lecture de l'effort normal, ou de la pression normale, permet de piloter les différents balayages afin de s'assurer que l'effort normal appliqué est sensiblement le même à chaque passage sur la surface à analyser. Un balayage le long d'une ligne d'analyse 60 permet l'obtention d'une courbe de frottement en fonction du temps f(t) 41 représentée à la figure 4. La courbe peut être décomposée par algorithme en trois parties. La première purement adhésive où l'élément frottant de la sonde exerce une contrainte pour cisailler le matériau et commencer le glissement. La seconde est une sorte de relaxation où le mouvement s'amorce libérant l'élément frottant de l'emprise des forces de surface. Et enfin la dernière est la phase dynamique où la sonde se met à se déplacer en frottant légèrement sur la surface. Chacune de ces parties de courbe est caractérisable par un paramètre mécanique qui sont la raideur (pente à l'origine), et les coefficients de friction statique et dynamique. Cet exemple d'analyse n'est pas limitatif.

Comme précisé précédemment, la mesure des efforts peut être influencée par la vitesse de balayage de la sonde sur la surface à analyser. Ce paramètre, mesuré grâce au dispositif de mesure de la vitesse, peut être pris en compte pour déterminer des valeurs d'analyse du toucher sensiblement indépendante de la vitesse de balayage et donc indépendante de l'utilisateur.

A titre d'exemple, l'impact d'un shampoing sur le coefficient de frottement d'un cheveu peut être mesuré en fonction du nombre de lavage.

## Revendications

1. Sonde pour la mesure quantitative du toucher d'une surface (20), comprenant :
- une enveloppe préhensible (1)
- un corps de contact (6) destiné à être en contact avec ladite surface sur une zone de sondage,
- des éléments de détection acoustique (5) pour détecter des bruits émis par le corps lors de son contact avec ladite zone de sondage,
- **caractérisée en ce que** le corps de contact (6) est un corps creux, que les éléments de détection acoustique (5) sont des premiers éléments de détection placés dans le corps creux et la sonde comprenant en outre des deuxièmes éléments de détection mécaniques (4, 7, 8) agencés pour mesurer l'effort normal ou la pression normale, et l'effort de frottement exercés par ladite surface sur ledit corps creux.

2. Sonde selon la revendication 1, dans laquelle les premiers éléments de détection acoustique comprennent un microphone maintenu dans ladite enveloppe préhensible, ce microphone comprenant une membrane (11) localisée à l'intérieur dudit corps creux.

3. Sonde selon l'une des revendications précédentes, dans laquelle les deuxièmes éléments de détection mécaniques comprennent au moins un capteur d'effort normal (4) adapté pour mesurer l'effort normal subi par ledit corps creux lors de son contact avec la zone de sondage.

4. Sonde selon l'une des revendications 1 et 2, dans laquelle les deuxièmes éléments de détection mécaniques comprennent au moins un capteur de pression normale adapté pour mesurer la pression normale subie par ledit corps creux lors de son contact avec la zone de sondage.

5. Sonde selon l'une des revendications précédentes, dans laquelle les deuxièmes éléments de détection mécaniques comprennent au moins un capteur d'effort de frottement (7, 8) adapté pour mesurer l'effort de frottement subi par ledit corps creux lors de son contact avec la zone de sondage.

6. Sonde selon l'une des revendications précédentes, comprenant une pièce de forme allongée (3) s'étendant entre deux extrémités, maintenue dans l'enveloppe préhensible et reliée à une de ses extrémités audit corps creux, ladite pièce étant adaptée pour transmettre les efforts normal et de frottement aux deuxièmes éléments de détection.

7. Sonde selon la revendication précédente, dans laquelle le capteur d'effort de frottement comprend un accéléromètre (7) et des jauges de contrainte (8) attachés à ladite pièce de forme allongée.

8. Sonde selon la revendication précédente, dans laquelle ladite pièce de forme allongée comprend deux ouvertures latérales (35) afin de former deux lames (36) de part et d'autre de ladite pièce de forme allongée, lesdites lames portant lesdites jauges de contrainte.

9. Sonde selon la revendication précédente, dans laquelle ladite pièce de forme allongée est formée d'un alliage métallique.

10. Sonde selon l'une des revendications précédentes, comprenant une diode (9) placée sur l'enveloppe préhensible et destinée à indiquer le sens de déplacement de ladite sonde lors de son contact avec la zone de sondage.

11. Sonde selon la revendication précédente, dans laquelle la diode est associée à une caméra optique pour former un dispositif de mesure de la vitesse de déplacement du corps creux sur la zone de sondage.

12. Sonde selon l'une des revendications précédentes, dans laquelle le corps creux est de forme sphérique.

13. Sonde selon l'une des revendications 1 à 11, dans laquelle le corps creux comprend une surface supérieure plane (6a), et une partie inférieure constituée d'une portion de cylindre (6b).

14. Sonde selon l'une des revendications 1 à 11, dans laquelle le corps creux comprend une surface supérieure (6a) et une surface inférieure (6b) planes et sensiblement parallèles, et présente la forme d'un parallélépipède.

15. Sonde selon l'une des revendications précédentes, dans laquelle le corps creux est en fibre de carbone.

16. Sonde selon l'une des revendications précédentes, comprenant des éléments de transmission (12) pour transmettre des données depuis les premiers et deuxièmes éléments de détection, ainsi que depuis le dispositif de mesure de la vitesse, vers une unité électronique de calcul (30).

17. Sonde selon la revendication précédente, dans laquelle l'unité électronique de calcul est adaptée pour transformer lesdites données en grandeurs simples permettant de quantifier le toucher de ladite zone de sondage.

18. Sonde selon la revendication précédente, dans laquelle un jeu (δ) est aménagé entre ledit corps creux et ladite enveloppe préhensible afin de permettre les déplacements normaux et tangentiels dudit corps creux.

19. Utilisation de la sonde selon les revendications 1 à 18 pour la mesure de l'impact sur les propriétés tribo-acoustiques d'un traitement appliqué à la surface de sondage.

## Patentansprüche

1. Sonde für die quantitative Messung der Berührung einer Oberfläche (20), umfassend:
- eine greifbare Hülle (1)
- einen Kontaktkörper (6), der ausgelegt ist, um mit der Oberfläche auf einem Untersuchungsbereich in Kontakt zu sein,
- akustische Nachweiselemente (5), um Geräusche nachzuweisen, die vom Körper bei seinem Kontakt mit dem Untersuchungsbereich ausgesendet werden,
- **dadurch gekennzeichnet, dass** der Kontaktkörper (6) ein hohler Körper ist, dass die akustischen Nachweiselemente (5) erste Nachweiselemente sind, die im hohlen Körper platziert sind, und die Sonde außerdem zweite mechanische Nachweiselemente (4, 7, 8) umfasst, die angeordnet sind, um die normale Beanspruchung oder den normalen Druck und die Reibungsbeanspruchung zu messen, die auf die Oberfläche des hohlen Körpers ausgeübt werden.

2. Sonde nach Anspruch 1, wobei die ersten akustischen Nachweiselemente ein Mikrophon umfassen, das in der greifbaren Hülle gehalten wird, wobei dieses Mikrophon eine Membran (11) umfasst, die sich im Inneren des hohlen Körpers befindet.

3. Sonde nach einem der vorhergehenden Ansprüche, wobei die zweiten mechanischen Nachweiselemente mindestens einen Sensor der normalen Beanspruchung (4) umfassen, der angepasst ist, um die normale Beanspruchung zu messen, der der hohle Körper bei seinem Kontakt mit dem Untersuchungsbereich unterzogen wird.

4. Sonde nach einem der Ansprüche 1 und 2, wobei die zweiten mechanischen Nachweiselemente mindestens einen Sensor des normalen Drucks umfassen, der angepasst ist, um den normalen Druck zu messen, dem der hohle Körper bei seinem Kontakt mit dem Untersuchungsbereich unterzogen wird.

5. Sonde nach einem der vorhergehenden Ansprüche, wobei die zweiten mechanischen Nachweiselemente mindestens einen Sensor der Reibungsbeanspruchung (7, 8) umfassen, der angepasst ist, um die Reibungsbeanspruchung zu messen, der der hohle Körper bei seinem Kontakt mit dem Untersuchungsbereich unterzogen wird.

6. Sonde nach einem der vorhergehenden Ansprüche, umfassend ein längliches Formstück (3), das sich zwischen zwei Enden erstreckt, gehalten in der greifbaren Hülle, und verbunden an einem seiner Enden mit dem hohlen Körper, wobei das Stück angepasst ist, um die normalen und Reibungsbeanspruchungen an die zweiten Nachweiselemente zu übertragen.

7. Sonde nach dem vorhergehenden Anspruch, wobei der Sensor der Reibungsbeanspruchung einen Beschleunigungsmesser (7) und Dehnmessstreifen (8) umfasst, die an das längliche Formstück befestigt sind.

8. Sonde nach dem vorhergehenden Anspruch, wobei das längliche Formstück zwei seitliche Öffnungen (35) umfasst, um zwei Schichten (36) auf beiden Seiten des länglichen Formstücks zu bilden, wobei die Schichten die Dehnmessstreifen tragen.

9. Sonde nach dem vorhergehenden Anspruch, wobei das längliche Formstück aus einer metallischen Legierung gebildet ist.

10. Sonde nach einem der vorhergehenden Ansprüche, umfassend eine Diode (9), die auf der greifbaren Hülle platziert und ausgelegt ist, um die Verschiebungsrichtung der Sonde bei ihrem Kontakt mit dem Untersuchungsbereich anzugeben.

11. Sonde nach dem vorhergehenden Anspruch, wobei die Diode mit einer optischen Kamera assoziiert ist, um eine Vorrichtung zum Messen der Geschwindigkeit der Verschiebung des hohlen Körpers auf dem Untersuchungsbereich zu bilden.

12. Sonde nach einem der vorhergehenden Ansprüche, wobei der hohle Körper eine sphärische Form aufweist.

13. Sonde nach einem der Ansprüche 1 bis 11, wobei der hohle Körper eine ebene obere Oberfläche (6a) und einen unteren Teil aufweist, der aus einem Zylinderteil (6b) besteht.

14. Sonde nach einem der Ansprüche 1 bis 11, wobei der hohle Körper eine obere Oberfläche (6a) und eine unter Oberfläche (6b) umfasst, die eben und im Wesentlichen parallel sind, und eine parallelepipede Form darstellt.

15. Sonde nach einem der vorhergehenden Ansprüche, wobei der hohle Körper aus Kohlenstofffaser ist.

16. Sonde nach einem der vorhergehenden Ansprüche, umfassend Übertragungselemente (12), um Daten von den ersten und zweiten Nachweiselementen ebenso wie von der Vorrichtung zum Messen der Geschwindigkeit, an die elektronische Recheneinheit (30) zu übertragen.

17. Sonde nach dem vorhergehenden Anspruch, wobei die elektronische Recheneinheit angepasst ist, um die Daten in einfache Größen umzuwandeln, die ermöglichen, die Berührung des Untersuchungsbereichs zu quantifizieren.

18. Sonde nach dem vorhergehenden Anspruch, wobei ein Spiel (δ) zwischen dem hohlen Körper und der greifbaren Hülle eingerichtet ist, um die normalen und tangentiellen Verschiebungen des hohlen Körpers zu ermöglichen.

19. Verwendung der Sonde nach Anspruch 1 bis 18 zur Messung der Wirkung einer Behandlung, angewendet auf die Untersuchungsoberfläche, auf die triboakustischen Eigenschaften.

## Claims

1. Sensor for the quantitative measuring of the feel of a surface (20), comprising:
- a grippable casing (1),
- a contact body (6) intended to contact said surface on a sensing zone,
- acoustic detection elements (5) to detect noises emitted by the body during the contact thereof with said sensing zone,
- **characterised in that** the contact body (6) is a hollow body, that the acoustic detection elements (5) are the first detection elements positioned in the hollow body and the sensor further comprising second mechanical detection elements (4, 7, 8) arranged to measure the normal force or the normal pressure, and the friction force exerted by said surface on said hollow body.

2. Sensor according to claim 1, wherein the first acoustic detection element comprise a microphone held in said grippable casing, this microphone comprising a membrane (11) located inside said hollow body.

3. Sensor according to one of the preceding claims, wherein the second mechanical detection elements comprise at least one normal force sensor (4) adapted to measure the normal force subjected by said hollow body during the contact thereof with the sensing zone.

4. Sensor according to one of the claims 1 and 2, wherein the second mechanical detection elements comprise at least one normal pressure sensor adapted to measure the normal pressure subjected by said hollow body during the contact thereof with the sensing zone.

5. Sensor according to one of the preceding claims, wherein the second mechanical detection elements comprise at least one friction force sensor (7, 8) adapted to measure the friction force subjected by said hollow body during the contact thereof with the sensing zone.

6. Sensor according to one of the preceding claims, comprising an elongated part (3) extending between two ends, held in the grippable casing and connected to one of the ends thereof to said hollow body, said part being adapted to transmit normal and friction forces to the two detection elements.

7. Sensor according to the preceding claim, wherein the friction force sensor comprising an accelerometer (7) and stress gauges (8) attached to said elongated part.

8. Sensor according to the preceding claim, wherein said elongated part comprises two lateral openings (35) in order to form two slides (36) on both sides of said elongated part, said slides holding said stress gauges.

9. Sensor according to the preceding claim, wherein said elongated part is formed from a metal alloy.

10. Sensor according to one of the preceding claims, comprising a diode (9) positioned on the grippable casing and intended to indicate the direction of movement of said sensor during the contact thereof with the sensing zone.

11. Sensor according to the preceding claim, wherein the diode is joined to an optical camera to form a device for measuring the movement speed of the hollow body on the sensing zone.

12. Sensor according to one of the preceding claims, wherein the hollow body is of a spherical form.

13. Sensor according to one of the claims 1 to 11, wherein the hollow body comprises an upper, flat surface (6a), and a lower part constituted of a cylinder portion (6b) .

14. Sensor according to one of the claims 1 to 11, wherein the hollow body comprises an upper surface (6a) and a lower surface (6b), flat and substantially parallel, and has the form of a parallelepiped.

15. Sensor according to one of the preceding claims, wherein the hollow body is made of carbon fibre.

16. Sensor according to one of the preceding claims, comprising transmission elements (12) to transmit data from the first and second detection elements, as well as from the speed measuring device, to an electronic calculation unit (30).

17. Sensor according to the preceding claim, wherein the electronic calculation unit is adapted to transform said data into simple sizes, enabling to quantify the feel of said sensing zone.

18. Sensor according to the preceding claim, wherein a unit (δ) is arranged between said hollow body and said grippable casing in order to enable normal and tangible movements of said hollow body.

19. Use of the sensor according to claims 1 to 18 for measuring the impact on the tribo-acoustic properties of a treatment applied to the sensing surface.
